# EUROPEAN PATENT APPLICATION

(11) **EP 2 792 669 A1**
(43) Date of publication of application: **22.10.2014**
(21) Application number: 13164361.1
(22) Date of filing: 18.04.2013
(51) Int. Cl.: C07C 213/08, C07C 217/80

(54) **Preparation of Bis-di(hetero)aryl-(hetero)arylamines**

(71) Applicant: Solvay SA, 1120 Bruxelles (BE)
(72) Inventor: The designation of the inventor has not yet been filed
(74) Representative: Mross, Stefan P.M.

(57) **Abstract**

The present invention relates to the preparation of certain bis-di(hetero)aryl-(hetero)arylamines via a process comprising at least two (hetero)aryl-(hetero)aryl coupling reactions. The target compounds are useful as building blocks for complex organic compounds, e.g. as building blocks for organic dye molecules in electrochemical and optoelectronic devices.

## Description

The present invention relates to a process for the preparation of certain bis-di(hetero)aryl-(hetero)arylamines useful as building blocks for complex organic compounds, e.g. as building blocks for organic dye molecules in electrochemical and optoelectronic devices.

Organic dye molecules are molecules sensible to the visible light. They form part of a dye-sensitized solar cell (DSSC), which is a class of photovoltaic cells.

Examples of useful organic dye molecules comprising a bis-di(hetero)aryl-(hetero)arylamine substructure have been described in European Patent Application No. 12188024.9, the contents of which are fully incorporated by reference herein.

Another example of an organic dye molecule containing a bis-di(hetero)aryl-(hetero)arylamine substructure is the compound Y123, the synthesis of which has been described in WO 2012/114315. The synthesis of Y123 proceeds via the bis-di(hetero)aryl-(hetero)arylamine substructure shown below :

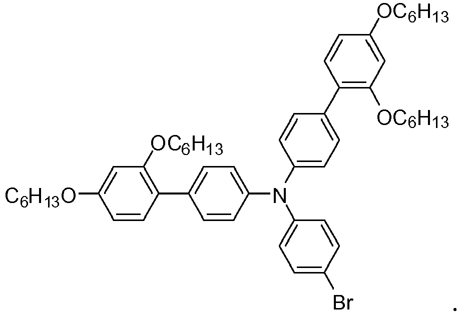

The synthesis of this building block has been described in WO 2012/114315 in seven synthetic steps from commercially available materials in a moderate overall yield.

Thus, there is a need for an improved process for the preparation of bis-di(hetero)aryl-(hetero)arylamines useful as building blocks for complex organic compounds.

Accordingly, it is an object of the present invention, amongst other objects to provide a process for the preparation of bis-di(hetero)aryl-(hetero)arylamines with an improved efficiency. An improved efficiency can be achieved for example by an improved overall yield, an improved purity of the products, the avoidance of certain high-cost and/or environmentally unfriendly materials, the avoidance of by-products, a reduction in energy-consumption of the process or by any combination of the above. Another object of the present invention is to provide a process for the preparation of bis-di(hetero)aryl-(hetero)arylamines with an improved versatility in terms of the product structures that can be prepared by the process.

Thus, the present invention relates in a first aspect to a process for the preparation of a compound of formula (I) : 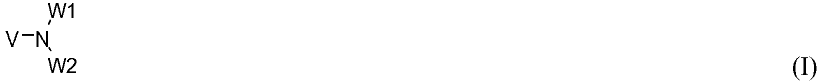 wherein V is a ring system comprising at least one aromatic ring and/or at least one heteroaromatic ring ; and wherein W1 and W2 are independently ring systems comprising at least two rings independently selected from aromatic rings and heteroaromatic rings said aromatic rings and/or heteroaromatic rings being connected to each other by a covalent single bond, comprising a first coupling reaction of a compound of general formula (II) : 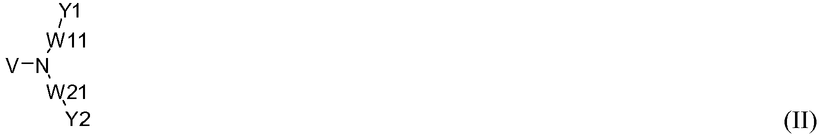 wherein V has the same meaning as described above, and W11 and W21 are independently ring systems comprising at least one aromatic ring and/or at least one heteroaromatic ring ; and wherein Y1 and Y2 are independently a halogen atom or an oxygen-containing leaving group ; with a compound of general formula (III) :

X1-Z1 (III)

wherein X1 is selected from the group consisting of B(OR₂), SnR₃ and SiR'₃,
wherein R is independently alkyl, alkylidene, aryl or heteroaryl and R' is independently alkyl, F, Cl, or OH ; and wherein Z1 is a ring system comprising at least one aromatic ring and/or at least one heteroaromatic ring ; to form a compound of general formula (IV) : 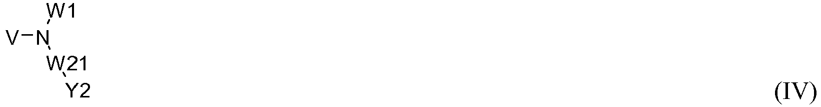 wherein V, W1, W21 and Y2 are as defined above ; and further comprising a second coupling reaction of the compound of general formula (IV) with a compound of general formula (V) :

X2-Z2 (V)

wherein X2 is selected from the group consisting of B(OR₂), SnR₃ and SiR'₃,
wherein R is independently alkyl, alkylidene, aryl or heteroaryl and R' is independently alkyl, F, Cl, or OH ; and wherein Z2 is a ring system comprising at least one aromatic ring and/or at least one heteroaromatic ring ;
or
comprising a coupling reaction of a compound of general formula (VI) : 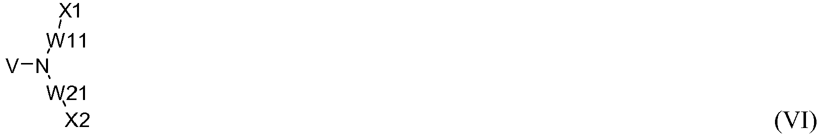 wherein V has the same meaning as described above, and W11 and W21 are independently a ring system comprising at least one aromatic ring and/or at least one heteroaromatic ring ; and
wherein X1 and X2 are independently selected from the group consisting of B(OR₂), SnR₃ and SiR'₃, wherein R is independently alkyl, alkylidene, aryl or heteroaryl and R' is independently alkyl, F, Cl, or OH ; with a compound of general formula (VII) :

Y1-Z1 (VII)

wherein Y1 is a halogen atom or an oxygen-containing leaving group and
wherein Z1 is a ring system comprising at least one aromatic ring and/or at least one heteroaromatic ring to form a compound of general formula (VIII) : 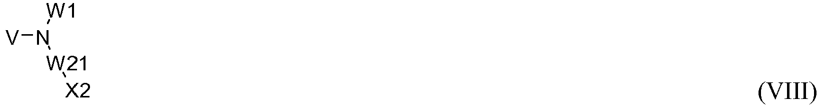 wherein V, W1, W21 and X2 are as defined above ; and further comprising a coupling reaction of the compound of general formula (VIII) with a compound of general formula (IX) :

Y2-Z2 (IX)

wherein Y2 is a halogen atom or an oxygen-containing leaving group, and
wherein Z2 is a ring system comprising at least one aromatic ring and/or at least one heteroaromatic ring.

The term "ring system" is intended to denote a moiety which comprises at least one aryl or heteroaryl ring.

The term "coupling reaction" is intended to denote a reaction in which a covalent bond is formed between two different ring systems.

The term "alkyl" is intended to denote in particular a linear or branched alkyl or a cycloalkyl group comprising from 1 to 20 carbon atoms, preferably 1, 2, 3, 4, 5, 6, 7, 8, 9 or 10 carbon atoms. Specific examples of such substituents are methyl, ethyl, n-propyl, isopropyl, n-butyl, isobutyl, tert-butyl, n-pentyl, isopentyl, n-hexyl, 2-hexyl, n-heptyl, n-octyl, cyclopropyl, cyclobutyl, cyclopentyl, cyclohexyl and cycloheptyl. The alkyl or cycloalkyl group can optionally be substituted, e.g. by halogen.

The term "aryl" is intended to denote a group which derives from an aromatic nucleus such as, in particular, a C6-C10 aromatic nucleus. Specific examples of such groups are phenyl, 1-tolyl, 2-tolyl, 3-tolyl, xylyl, 1-naphthyl and 2-naphthyl, in particular phenyl or naphthyl. The aryl group can optionally be substituted, e.g. by halogen, alkyl, or cycloalkyl.

The term "heteroaryl" is intended to denote in particular a cyclic aromatic group made up of 3, 4, 5, 6, 7 or 8 atoms, at least one of which is a hetero atom. The hetero atom is often chosen from B, N, O, Si, P and S. It is more often chosen from N, O and S. Specific examples of such heteroaryls are pyridine, pyrimidine, thiophene, thiazole, quinoline, isoquinoline, isoxazole, pyrazole, imidazole, furan, dioxane. The heteroaryl group can optionally be substituted, e.g. by halogen, alkyl, or cycloalkyl.

The term "alkylidene" is intended to denote in particular a C2 to C7 alkylene group, including a vinylidene group, wherein the alkylidene group preferably comprises a bridge of 2, 3, 4, 5, or 6 carbon atoms, more preferably 2 carbon atoms. The alkylidene group can optionally be substituted, e.g. by halogen, alkyl, or cycloalkyl.

The term "oxygen-containing leaving group" is intended to denote a group that contains at least one oxygen atom and that is detached from the rest of a molecule during a reaction. Specific examples for suitable oxygen-containing leaving groups are mesylate (CH₃SO₃⁻), tosylate (4-CH₃(C₆H₄)SO₃⁻), besylate (C₆H₅SO₃⁻), triflate (CF₃SO₃⁻), hydroxyl (OH⁻) or alkoxy groups (alkyl-O⁻).

In a preferred embodiment the invention related to a process comprising a coupling reaction of a compound of general formula (II) : 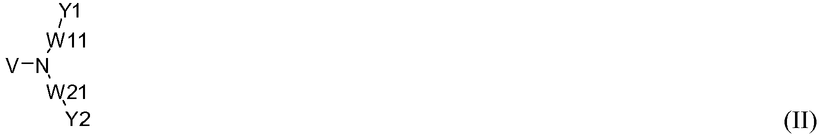 wherein V is a ring system comprising at least one aromatic ring and/or at least one heteroaromatic ring ; and wherein W11 and W21 are independently ring systems comprising at least one aromatic ring and/or at least one heteroaromatic ring ; and wherein Y1 and Y2 are independently a halogen atom or an oxygen-containing leaving group ; with a compound of general formula (III) :

X1-Z1 (III)

wherein X1 is selected from the group consisting of B(OR₂), SnR₃ and SiR'₃,
wherein R is independently alkyl, alkylidene, aryl or heteroaryl and R' is independently alkyl, F, Cl, or OH ; and wherein Z1 is a ring system comprising at least one aromatic ring and/or at least one heteroaromatic ring ; to form a compound of general formula (IV) : 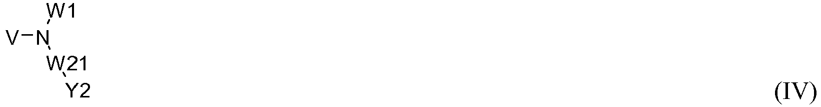 wherein V, W1, W21 and Y2 are as defined above ; and further comprising a coupling reaction of the compound of general formula (IV) with a compound of general formula (V) :

X2-Z2 (V)

wherein X2 is selected from the group consisting of B(OR₂), SnR₃ and SiR'₃,
wherein R is independently alkyl, alkylidene, aryl or heteroaryl and R' is independently alkyl, F, Cl, or OH ; and wherein Z2 is a ring system comprising at least one aromatic ring and/or at least one heteroaromatic ring.

In another preferred embodiment the invention relates to a process wherein X is B(OR₂), wherein R is independently alkyl, alkylidene, aryl or heteroaryl, more preferably X is a boronic acid alkylidene ester, even more preferably X is boronic acid tetramethylethylene ester.

In yet another preferred embodiment the invention relates to a process wherein the coupling reaction is carried out in the presence of a transition-metal catalyst.

Suitable examples of transition metal catalysts are
tris(dibenzylideneacetone)dipalladium(0), Pd(Ph)₄, Pd(OAc)₂ or PdCl₂. Most preferred is Pd(Ph)₄.

In still another preferred embodiment the invention relates to a process wherein Y1 and Y2 are independently selected form Br and I, more preferably Y1 and Y2 are I.

The coupling reaction according to the process of the invention may advantageously be carried out in one chemical step, i.e. the first and the second coupling reaction are carried out without isolation of the intermediate product after the first coupling step and/or without transferring the reaction mixture to a different vessel after the first coupling reaction and/or without adding further reagents or reactants after the first coupling reaction. Suitably, the first and the second coupling step are carried out in the same reaction vessel. Alternatively, the intermediate product after the first coupling reaction is isolated, optionally purified, and subjected to the second coupling reaction in the same or in a different reaction vessel. Preferably, the first coupling reaction and the second coupling reaction are carried out in one chemical step.

In still another preferred embodiment the invention relates to a process wherein Z1 and Z2 are the same, more preferably Z1 and Z2 are a substituted phenyl ring, even more preferably Z1 and Z2 are an alkoxy-substituted phenyl ring, and most preferably Z1 and Z2 are 2,4-dihexyloxyphenyl.

In still another preferred embodiment the invention relates to a process wherein W11 and W21 are 1,4-phenylidine.

In still another preferred embodiment the invention relates to a process wherein V is a substituted phenyl ring, more preferably V is a phenyl ring substituted with a halogen atom, most preferably V is 4-bromophenyl.

In still another preferred embodiment the invention relates to a process wherein W1 and W2 are

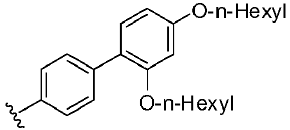

In still another preferred embodiment the invention relates to a process wherein the compound of formula (I) is

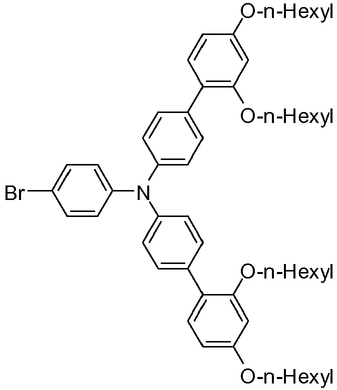

In another aspect the invention relates to a process for the preparation of an organic dye molecule comprising the process according to any one of the embodiments of the first aspect of the invention and a process to further convert the compound of formula (I) into an organic dye molecule. In a preferred embodiment of this aspect of the invention the process to further convert the compound of formula (I) into an organic dye molecule comprises a further coupling reaction. Suitable examples of processes to further convert the compound of formula (I) into an organic dye molecule have been described in European Patent Application No. 12188024.9.

The process for the preparation of an organic dye molecule according to the invention can be suitably applied to the manufacture of organic dye molecules suitable for use in organic solar cells or organic light emitting devices, preferably organic solar cells.

The coupling reactions according to the present invention are generally performed in presence of a solvent. Examples of suitable solvents are hydrocarbons (e.g. toluene), alcohols (e.g. tert-butanol), acetonitrile, tetrahydrofurane, dioxane, dimethylformamide, dimethylacetamide or mixtures thereof. The reaction is usually performed under inert atmosphere, i.e. under an atmosphere which is virtually free of oxygen. The solvent is usually degassed by passing nitrogen through the reaction mixture or by performing at least one freezing-thawing cycle under vacuum.

The coupling reactions according to the present invention can be carried out in the presence of one or more phosphorus-containing ligands and/or one or more bases. Suitable examples of phosphorus-containing ligands are triphenylphosphine and 2-dicyclohexylphosphino-2',4',6'-triisopropylbiphenyl. Suitable examples for bases are hydroxides, phosphates, alkoxides and carbonates of elements of the group 1 or group 2 elements, e.g. sodium hydroxide, cesium carbonate, potassium phosphate or potassium tert-butoxide. Advantageously, these bases can be used in the form of an aqueous solution, more particularly a solution in water.

The coupling reactions according to the present invention can be carried out at a temperature in the range from 0 to 150 °C, preferably in the range from 50 to 100 °C.

Coupling reactions wherein X is SnR3 are preferably carried out in dehydrated solvents, more preferably in dehydrated dioxane. Also preferably, SnR3 is tributyltin or trimethyltin, more preferably tributyltin. Advantageously, LiCl can be added to the reaction mixture.

Coupling reactions wherein X is SiR'3 are preferably carried out in the presence of fluoride ions, more preferably in the presence of tetrabutylammonium fluoride. Also preferably, SiR'₃ is SiMe₃, Si(iso-Pr)₃ or SiEtCl₂.

The compounds prepared by the process according to the invention may be further isolated, for instance by column chromatography or by recrystallisation. While preferred embodiments of this invention have been shown and described, modifications thereof can be made by one skilled in the art without departing from the spirit or teaching of this invention. The embodiments described herein are exemplary only and are not limiting. Many variations and modifications of systems and methods are possible and are within the scope of the invention. Accordingly, the scope of protection is not limited to the embodiments described herein, but is only limited by the claims that follow, the scope of which shall include all equivalents of the subject matter of the claims.

### Examples

List of abbreviations :
RT- room temperature
Pd₂dba₃ - tris(dibenzylideneacetone)dipalladium(0)
X-Phos - 2-dicyclohexylphosphino-2',4',6'-triisopropylbiphenyl
DCM - dichloromethane

### 2-(2,4-bis(hexyloxy)phenyl)-4,4,5,5-tetramethyl-1,3,2-dioxaborolane

In a 100 mL single-neck round-bottom flask, 8.56 g 1-chloro-2,4-bis(hexyloxy)benzene (27.36 mmol), 10.42 g bis(pinacolato)diboron (41.03 mmol), and 8.06 g potassium acetate (82.12 mmol) were dissolved in 60 mL dioxane. This solution was degassed for 20 minutes with a stream of _{N}2, after which 10 mg Pd₂dba₃ and 20 mg X-Phos were added simultaneously, in one batch. The reaction was then brought to 95°C for 10 h. Afterwards, the reaction was cooled to RT, and plugged through a thin pad of MgSO₄ with DCM. The crude product was purified by gradient silica gel chromatography : 100 % hexanes to 20:80 hexanes/DCM. 6.23 g (70 % yield) of a pure clear oil was obtained. ¹H NMR (400 MHz, D₂-DCM) δ 7.54 (d, *J*= 8.2 Hz, 1H), 6.46 (dd, *J*= 8.2, 2.2 Hz, 1H), 6.40 (d, *J*= 2.2 Hz, 1H), 3.96 (dt, *J*= 14.4, 6.4 Hz, 4H), 1.85 - 1.72 (m, 4H), 1.61 - 1.42 (m, 4H), 1.41 - 1.34 (m, 8H), 1.32 (s, 12H), 0.99 - 0.86 (m, 6H) ppm. ¹³C NMR (100 MHz, CD₂Cl₂) δ = 165.58,163.11, 137.71, 129.65, 105.10, 99.20, 83.17, 82.81, 68.20, 67.88, 31.66, 31.58, 29.32, 29.20, 25.68, 25.67, 24.83, 24.64, 22.71, 22.60, 13.88, 13.80 ppm.

### 4-bromo-N,N-bis(4-iodophenyl)aniline

A mixture of 4-bromo-*N,N*-diphenylaniline (3.2 g, 9.87 mmol), KI (2.23 g, 13.42 mmol), and KIO₃ (1.415 g, 6.6 mol) was stirred at 85°C in 50 ml HOAc for 12 h. After cooling to RT, aq. NaHSO₃ (50 mL) was added and the reaction mixture was stirred for 20 min. The solution was extracted with CH₂Cl₂ (3 x 50 mL) and the organic layer was dried with MgSO₄. After removal of the solvent, the crude product was purified by recrystallization (DCM/MeOH) to afford the target product as a white solid (5.57g, 96 %). ¹H NMR (400 MHz, CDCl₃) δ 7.47 (m, 4 H), 7.28 (m, 2 H), 6.86 (m, 2 H), 6.74 (m, 4 H) ppm. ¹³C NMR (101 MHz, CDCl₃) δ 146.7, 145.9, 138.5, 132.6, 126.0, 116.3, 86.5 ppm.

### N-(2',4'-bis(hexyloxy)biphenyl-4-yl)-N-(4-bromophenyl)-2',4'-bis(hexyloxy)biphenyl-4-amine

2-(2,4-bis(hexyloxy)phenyl)-4,4,5,5-tetramethyl-1,3,2-dioxaborolane (1 g, 2.473 mmol) and 4-bromo-N,N-bis(4-iodophenyl)aniline (647 mg, 1.124 mmol) were dissolved in toluene (30 ml). An aqueous K₂CO₃ solution (2.764 g in 10 ml H₂O) was added. Nitrogen was bubbled through the biphasic system for 20 min. Then Pd(PPh₃)₄ (40 mg) was added and the mixture was heated to 85°C and kept at that temperature for 24 hours. Purification by silica gel chromatography using hexanes/DCM (3:1) yielded 818 mg (83 %) as a light yellow oil. ¹H NMR (400 MHz, CD₂Cl₂) δ 7.50 (m, 4 H), 7.40 (m, 2 H), 7.28 (d, 2 H, *J*= 8.4 Hz), 7.16 (m, 4 H), 7.08 (m, 2 H), 4.01 (m, 8 H), 1.82 (m, 8 H), 1.44 (m, 28 H), 0.97 (m, 14 H) ppm. ¹³C NMR (101 MHz, CD₂Cl₂) δ 159.8, 157.0, 147.2, 145.5, 133.6, 132.0, 130.7, 130.3, 125.0, 123.8, 122.6, 114.3, 105.4, 100.2, 99.8, 68.0, 31.6, 29.2, 25.6, 22.7, 13.9.

## Claims

1. A process for the preparation of a compound of formula (I) : 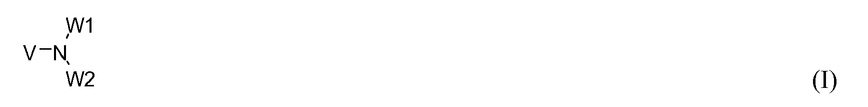 wherein V is a ring system comprising at least one aromatic ring and/or at least one heteroaromatic ring ; and
wherein W1 and W2 are independently ring systems comprising at least two rings independently selected from aromatic rings and heteroaromatic rings said aromatic rings and/or heteroaromatic rings being connected to each other by a covalent single bond,
comprising a first coupling reaction of a compound of general formula (II) : 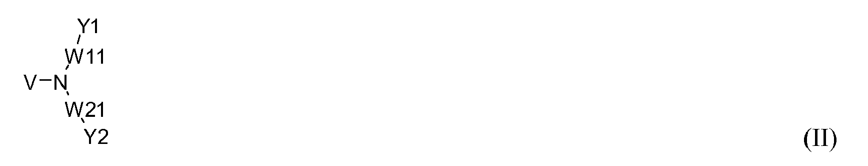 wherein V has the same meaning as described above, and W11 and W21 are independently ring systems comprising at least one aromatic ring and/or at least one heteroaromatic ring ; and
wherein Y1 and Y2 are independently a halogen atom or an oxygen-containing leaving group ;
with a compound of general formula (III) :
X1-Z1 (III)
wherein X1 is selected from the group consisting of B(OR₂), SnR₃ and SiR'₃, wherein R is independently alkyl, alkylidene, aryl or heteroaryl and R' is independently alkyl, F, Cl, orOH ; and
wherein Z1 is a ring system comprising at least one aromatic ring and/or at least one heteroaromatic ring ;
to form a compound of general formula (IV) : 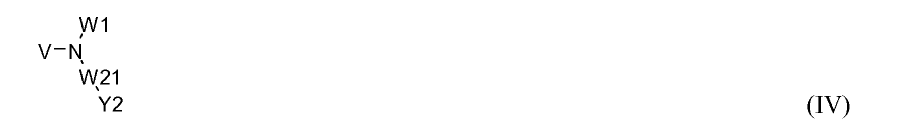 wherein V, W1, W21 and Y2 are as defined above ; and
further comprising a second coupling reaction of the compound of general formula (IV) with a compound of general formula (V) :
X2-Z2 (V)
wherein X2 is selected from the group consisting of B(OR₂), SnR₃ and SiR'₃, wherein R is independently alkyl, alkylidene, aryl or heteroaryl and R' is independently alkyl, F, Cl, or OH ; and
wherein Z2 is a ring system comprising at least one aromatic ring and/or at least one heteroaromatic ring ;
or
comprising a coupling reaction of a compound of general formula (VI) : 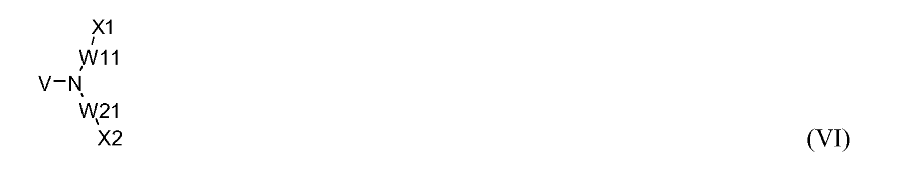 wherein V has the same meaning as described above, and W11 and W21 are independently a ring system comprising at least one aromatic ring and/or at least one heteroaromatic ring ; and
wherein X1 and X2 are independently selected from the group consisting of B(OR₂), SnR₃ and SiR'₃, wherein R is independently alkyl, alkylidene, aryl or heteroaryl and R' is independently alkyl, F, Cl, or OH ;
with a compound of general formula (VII) :
Y1-Z1 (VII)
wherein Y1 is a halogen atom or an oxygen-containing leaving group and
wherein Z1 is a ring system comprising at least one aromatic ring and/or at least one heteroaromatic ring
to form a compound of general formula (VIII) : 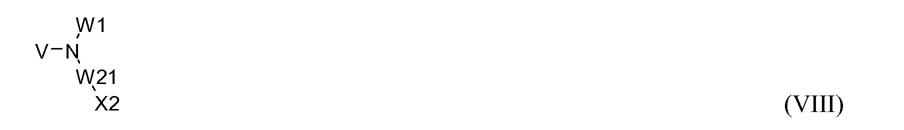 wherein V, W1, W21 and X2 are as defined above ; and
further comprising a coupling reaction of the compound of general formula (VIII) with a compound of general formula (IX) :
Y2-Z2 (IX)
wherein Y2 is a halogen atom or an oxygen-containing leaving group, and
wherein Z2 is a ring system comprising at least one aromatic ring and/or at least one heteroaromatic ring.

2. The process of claim 1 comprising a coupling reaction of a compound of general formula (II) : 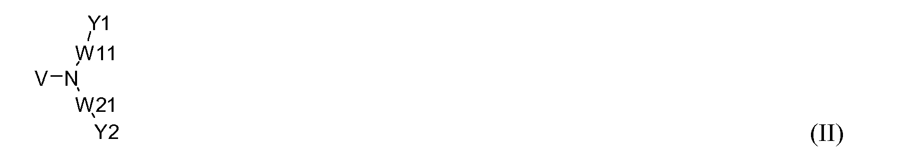 wherein V is a ring system comprising at least one aromatic ring and/or at least one heteroaromatic ring ; and
wherein W11 and W21 are independently ring systems comprising at least one aromatic ring and/or at least one heteroaromatic ring ; and
wherein Y1 and Y2 are independently a halogen atom or an oxygen-containing leaving group ;
with a compound of general formula (III) :
X1-Z1 (III)
wherein X1 is selected from the group consisting of B(OR₂), SnR₃ and SiR'₃,
wherein R is independently alkyl, alkylidene, aryl or heteroaryl and R' is independently alkyl, F, Cl, or OH ; and
wherein Z1 is a ring system comprising at least one aromatic ring and/or at least one heteroaromatic ring ;
to form a compound of general formula (IV) : 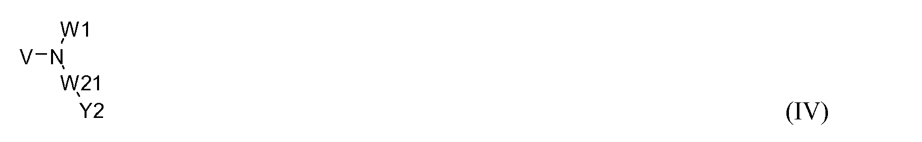 wherein V, W1, W21 and Y2 are as defined above ; and
further comprising a coupling reaction of the compound of general formula (IV) with a compound of general formula (V) :
X2-Z2 (V)
wherein X2 is selected from the group consisting of B(OR₂), SnR₃ and SiR'₃,
wherein R is independently alkyl, alkylidene, aryl or heteroaryl and R' is independently alkyl, F, Cl, or OH ; and
wherein Z2 is a ring system comprising at least one aromatic ring and/or at least one heteroaromatic ring.

3. The process of claim 1 or 2 wherein X is B(OR₂), wherein R is independently alkyl, alkylidene, aryl or heteroaryl, preferably X is a boronic acid alkylidene ester, more preferably X is boronic acid tetramethylethylene ester.

4. The process of any one of the claims 1 to 3 wherein the coupling reaction is carried out in the presence of a transition-metal catalyst.

5. The process of claim 4 wherein the transition-metal catalyst is Pd(Ph)₄.

6. The process of any one of the claims 1 to 5 wherein Y1 and Y2 are independently selected form Br and I, preferably Y1 and Y2 are I.

7. The process of any one of the claims 1 to 6 wherein the first coupling reaction and the second coupling reaction are carried out in one chemical step.

8. The process of any one of the claims 1 to 7 wherein Z1 and Z2 are the same, preferably Z1 and Z2 are a substituted phenyl ring, more preferably Z1 and Z2 are an alkoxy-substituted phenyl ring, most preferably Z1 and Z2 are 2,4-dihexyloxyphenyl.

9. The process of any one of the claims 1 to 8 wherein W11 and W21 are 1,4-phenylidine.

10. The process of any one of the claims 1 to 9 wherein V is a substituted phenyl ring, preferably V is a phenyl ring substituted with a halogen atom, more preferably V is 4-bromophenyl.

11. The process of any one of the claims 1 to 10 wherein W1 and W2 are

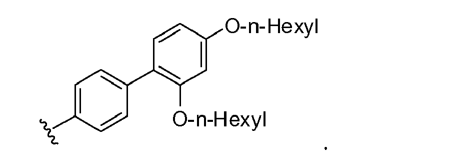

12. The process of any one of claims 1 to 11 wherein the compound of formula (I) is

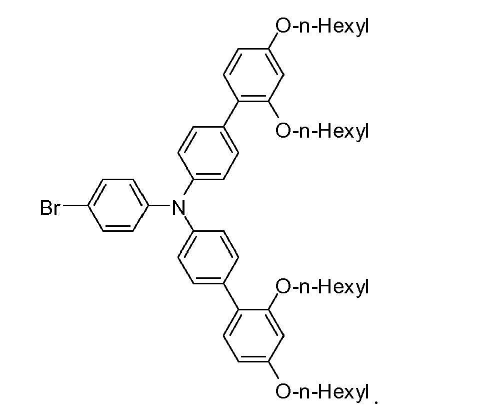

13. A process for the preparation of an organic dye molecule for organic solar cells comprising
a) the process according to any one of the claims 1 to 12 and
b) a process to further convert the compound of formula (I) into an organic dye molecule for organic solar cells.

14. The process of claim 13 wherein the process of b) comprises a further coupling reaction.

15. The process according to claim 13 comprising the process according to claim 12.
